# EUROPEAN PATENT APPLICATION

(11) **EP 4 197 543 A1**
(43) Date of publication of application: **21.06.2023**
(21) Application number: 20949719.7
(22) Date of filing: 17.08.2020
(51) Int. Cl.: A61K 31/7034, A61K 31/4178, A61K 31/38, A61P 9/12, A61P 3/10, A61P 3/06, A61P 3/04, A61P 3/08

(54) **COMPOSITION AND USE OF SGLT-2 INHIBITOR AND ANGIOTENSIN RECEPTOR BLOCKERS**

(71) Applicant: Beijing Creatron Institute of Pharmaceutical Research Co., Ltd., Beijing 102200 (CN)
(72) Inventor: JIA, Huijuan, Beijing 102200 (CN); SHI, Hongwei, Beijing 102200 (CN); ZHANG, Jiayan, Beijing 102200 (CN); REN, Xiaohui, Beijing 102200 (CN)
(74) Representative: IPrime Rentsch Kaelin AG
(86) International application number: PCT/CN2020/109533
(87) International publication number: WO 2022/036506

(57) **Abstract**

The present invention relates to the field of medicine, in particular to a composition and use of an SGLT-2 inhibitor and angiotensin receptor blockers, and specifically to a fixed-dose compound composition and use of a sodium-glucose cotransporter 2 (SGLT-2) inhibitor and angiotensin receptor blockers (ARBs). The fixed-dose compound composition is used for the treatment of patients with diabetes and hypertension, and also benefits patients with chronic kidney diseases, preventing or delaying the deterioration of renal failure in patients with chronic kidney diseases (CKD), and preventing cardiovascular (CV) death or death from kidney diseases. In addition, the fixed-dose compound composition has good synergy and good stability, is convenient to carry or administer, significantly reduces the cost of medication, also avoids the risk of excessive and missing administration, and improves the compliance of patients with medication.

## Description

### FIELD

The present disclosure relates to the field of medicine, and in particular to a composition of an SGLT-2 inhibitor and an angiotensin receptor blocker and use thereof.

### BACKGROUND

Hypertension is a major risk factor for cardiovascular disease, especially for patients still with diabetes considering their synergistic harm on cardiovascular system. An abnormally elevated blood sugar and insulin resistance in patients with type 2 diabetes are independent risk factors for the onset of multiple cardiovascular diseases including hypertension. Meanwhile, controlling blood pressure and avoiding excessive fluctuations of blood pressure in patients with type 2 diabetes have a significant effect in preventing or controlling long-term cardiovascular complications in patients with diabetes. In a case that these risk factors are effectively controlled, the benefits are very obvious. The relevant epidemiological research shows that in recent years, the prevalence of hypertension and diabetes in China has been increasing year by year. 32.9% patients with hypertension in cardiology department also had diabetes, while 58.9% patients with diabetes in endocrinology department also had hypertension. In the general population, 45.3% patients suffer from both hypertension and diabetes. The prevalence of hypertension in patients with diabetes is significantly increased, about twice that in people without diabetes. In the United States, the prevalence of diabetes is the highest among American adults, and about half of patients with diabetes also suffer from hypertension, which undoubtedly increases the cardiovascular burden of patients with diabetes. Diabetes associated with hypertension further increases the risks of left ventricular wall hypertrophy, proteinuria and renal function damage in patients with type 2 diabetes, increases the incidence of adverse cardiovascular events in patients with cardiovascular disease, and increases the economic burden of such patients. Hypertension associated with type 2 diabetes seriously damages the heart, brain, kidneys and other important target organs and the vascular system of the body of patients, leading to complications such as coronary atherosclerotic heart disease, cardiac insufficiency, central nervous system vascular ischemia or hemorrhagic stroke, renal insufficiency and even uremia, which seriously affect the health and quality of life of such patients. Therefore, in a patient suffering from both hypertension and diabetes, controlling blood sugar and blood pressure simultaneously can more effectively reduce the occurrence of complications to reduce the risk of death.

Hypertension and diabetes have high morbidity and mortality, and thus relevant treatment should be carried out in time after diagnosis. However, treating patients with diabetes associated with hypertension brings certain challenges to clinical treatment. There are many clinical methods to treat diabetes with hypertension, among which drug therapy is the most common. However, most patients with diabetes associated with hypertension are elderly, and the drug therapy requires taking drug for life. The treatment of such patients needs to improve the therapeutic effect as much as possible on the one hand, and minimize the occurrence of adverse reactions on the other hand, so as to increase the patient's treatment compliance and obtain a satisfactory treatment effect. Irbesartan (active ingredient A) is an angiotensin receptor blocker with high selectivity, which does not produce agonistic effects while functioning to competitively antagonize, and aims to lower blood pressure by inhibiting the release of aldosterone from the adrenal glands and dilating blood vessels. Irbesartan protects the kidneys by inhibiting the proliferation of cells in mesangium, reducing the content of basal protein in the body and improving the excretion rate of urinary albumin in the body. Irbesartan is used to treat essential hypertension and type 2 diabetic nephropathy associated with hypertension. Clinical studies have proved that irbesartan can benefit the kidneys of patients with hypertension associated with type 2 diabetes.

Dapagliflozin (active ingredient B) is a sodium-glucose co-transporter 2 (SGLT-2) inhibitor with extremely high selectivity and specificity. Upon entering the body, it controls blood sugar by blocking the absorption of glucose in the body by the convoluted tubules, increasing the excretion of the body and reducing the level of glycosylated hemoglobin. What is more exciting is that SGLT-2 inhibitors also show a good prospect in lowering blood pressure, which provides a new and unique idea for the treatment of diabetes and hypertension. Recent clinical trials of SGLT-2 inhibitors have demonstrated that when the systolic blood pressure of patients with diabetes decreased by 4 mmHg, their diastolic blood pressure only decreased by 1.6 mmHg. However, in a case of using the common antihypertensive drugs for monotherapy, when the systolic blood pressure decreased by 9.1 mmHg, the diastolic blood pressure decreased by 5.5 mmHg. Too much reduction in diastolic blood pressure will lead to the risk of cardiac ischemia. In 2019, dapagliflozin was granted fast track designation by the US Food and Drug Administration (FDA) to delay the deterioration of renal failure in patients with chronic kidney disease (CKD) and prevent cardiovascular (CV) and renal death, including CKD patients with or without type 2 diabetes (T2D).

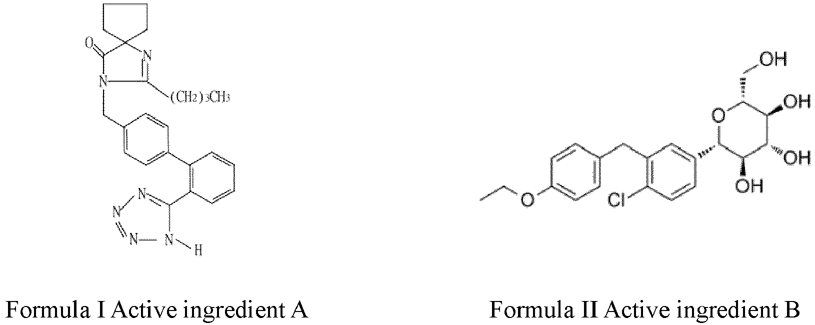

For patients with diabetes associated with hypertension, administering an SGLT-2 inhibitor together with an angiotensin receptor antagonist can reduce blood pressure and blood sugar at the same time, benefit the kidneys and cardiovascular system, prevent or delay the deterioration of renal failure in patients with chronic kidney disease (CKD) and prevent cardiovascular (CV) and renal death. At present, the single formulations of the two are given to patients at the same time in clinical practice, causing disadvantages of inconvenience to carry or administer, poor compliance, risks of excessive or missing administration of one drug, potential safety hazards and high drug cost. There is no compound formulation of irbesartan and dapagliflozin on the market or related patents of the compound formulation currently.

### SUMMARY

In view of this, the present disclosure provides a composition of an SGLT-2 inhibitor and an angiotensin receptor blocker and use thereof. The composition is used for the treatment of diabetes with hypertension, while benefiting the patient's kidney or cardiovascular system and reducing the risk of cardiovascular and renal complications in patients with type 2 diabetes. The composition according to the present disclosure is convenient to carry or administer, reduces medication cost, avoids the risk of excessive and missing administration, and improves the medication compliance of patients.

In order to achieve the above-mentioned purpose of the present disclosure, the following technical solutions are provided according to the present disclosure.

A composition comprising an SGLT2 inhibitor and an angiotensin II receptor antagonist is provided according to the present disclosure.

In some specific embodiments of the present disclosure, the SGLT-2 inhibitor is selected from the group consisting of dapagliflozin, canagliflozin, empagliflozin, ipragliflozin, luseogliflozin, tofogliflozin, ertugliflozin, janagliflozin, bexagliflozin, sotagliflozin, henagliflozin, tianagliflozin, remogliflozin, alligliflozin, remogliflozin etabonate, (1R,2S,3S,4R,5S)-5-(4-chloro-3-(4-ethoxybenzyl)phenyl)-1-((R)-1-hydroxyethyl)-6,8-dioxabicyc lo[3.2.1]octane-2,3,4-triol, and a mixture thereof.

In some specific embodiments of the present disclosure, the angiotensin II receptor antagonist is selected from the group consisting of irbesartan, candesartan, valsartan, telmisartan, losartan, iprasartan, olmesartan, azilsartan, and a mixture thereof.

In some specific embodiments of the present disclosure, the SGLT-2 inhibitor is selected from the group consisting of dapagliflozin, a pharmaceutically acceptable salt, ester, cocrystal, complex or solvate of dapagliflozin, and a mixture thereof; and the angiotensin II receptor antagonist is selected from the group consisting of irbesartan, a pharmaceutically acceptable salt or solvate of irbesartan, and a mixture thereof.

In some specific embodiments of the present disclosure, a weight ratio of the SGLT-2 inhibitor to the angiotensin II receptor antagonist is 1:960-300:1, preferably 1:240-75:1, more preferably 1:120-1:7.5, more preferably (8-300):(2-300), and most preferably 8:300, 20:12.3, 40:12.3, 50:4.1, 50:2, 44.8:3.7, 32.5:2.7, 150:12.3, 150:6.15, 75:12.3, 300:6.15 or 300:12.3.

On the basis of the above, use of the composition in the manufacture of a medicament for treating essential hypertension, type 2 diabetic nephropathy associated with hypertension, diabetes, insulin resistance, hyperglycemia, hyperinsulinemia, elevated level of fatty acid or glycerol in blood, hyperlipidemia, dyslipidemia, obesity, or complications of diabetes is further provided according to the present disclosure.

A drug comprising the composition and a pharmaceutically acceptable adjuvant is further provided according to the present disclosure.

In some specific embodiments of the present disclosure, the adjuvant is selected from the group consisting of a filler, binder, disintegrant, antiadherent, adsorbent, glidant, lubricant, surfactant, chelating agent, colorant, taste masking agent, and a mixture thereof.

In some specific embodiments of the present disclosure, the drug is in a dosage form selected from the group consisting of a tablet, capsule, granule, suspension, film, and a mixture thereof, preferably a tablet, dry suspension, capsule or granule.

In some embodiments of the present disclosure, the drug is a tablet, granule or capsule, which comprises:
a) an SGLT-2 inhibitor and an angiotensin II receptor blocker;
b) optional one or more fillers;
c) optional one or more binders;
d) optional one or more disintegrants;
e) optional one or more antiadherents or adsorbents;
f) optional one or more glidants or lubricants;
g) optional one or more surfactants;
h) optional one or more colorants;
i) optional one or more flavoring agents.

Wherein, the SGLT-2 inhibitor is selected from the group consisting of dapagliflozin, canagliflozin, empagliflozin, ipragliflozin, luseogliflozin, tofogliflozin, ertugliflozin, janagliflozin, bexagliflozin, sotagliflozin, henagliflozin, tianagliflozin, remogliflozin, alligliflozin, remogliflozin etabonate, (1R,2S,3S,4R,5S)-5-(4-chloro-3-(4-ethoxybenzyl)phenyl)-1-((R)-1-hydroxyethyl)-6,8-dioxabicyc lo[3.2.1]octane-2,3,4-triol, and a mixture thereof. Dapagliflozin is preferred. Dapagliflozin or a pharmaceutically acceptable salt, ester, cocrystal, complex or solvate thereof is preferred.

The angiotensin II receptor antagonist is selected from the group consisting of irbesartan, candesartan, valsartan, telmisartan, losartan, iprasartan, olmesartan, and azilsartan. Irbesartan or a pharmaceutically acceptable salt, cocrystal, complex or solvate thereof is preferred.

The diluent in the composition according to the present disclosure may be one or more compounds that can provide a desired tablet volume. The ideal diluent includes, but is not limited to, microcrystalline cellulose, lactose, mannitol, erythritol, maltitol, sorbitol, trehalose, sucrose, white sugar, glucose, fructose, corn starch, wheat starch, dextrin, licorice powder, calcium phosphate, calcium hydrogen phosphate, calcium carbonate, calcium citrate, calcium lactate, magnesium oxide, magnesium hydroxide, calcium oxide, magnesium carbonate, potassium carbonate, zinc carbonate, titanium oxide, silicic anhydride, magnesium silicate, calcium silicate, sodium bicarbonate, magnesium sulfate, calcium sulfate, and silicon dioxide.

The binder in the composition according to the present disclosure may be one or more compounds that enable the main drug and adjuvant to become desired free-flowing particles. The ideal binder includes, but is not limited to, hydroxypropyl cellulose, hydroxypropyl methylcellulose, white sugar, gelatin, starch, Arabic gum, astragalus gum, carboxymethyl cellulose, polyvinylpyrrolidone, methyl cellulose, partially pregelatinized starch, pregelatinized starch, polyvinyl alcohol, sodium alginate, pullulan and glycerol.

The disintegrant in the composition according to the present disclosure may be one or more compounds that facilitate the disintegration of the composition upon contact with an aqueous medium. The preferred disintegrant includes, but is not limited to, corn starch, partially pregelatinized starch, hydroxypropyl starch, carboxymethyl cellulose, sodium carboxymethyl cellulose, calcium carboxymethyl cellulose, sodium carboxymethyl starch, low-substituted hydroxypropyl cellulose, cross-linked sodium carboxymethyl cellulose, and crospovidone.

The antiadherent or adsorbent in the composition according to the present disclosure refers to one or more compounds that can reduce the viscosity of the formulation or adsorb the liquid component in the formulation to prevent it from adhering to a metal surface. The antiadherent or adsorbent includes, but is not limited to, silicon dioxide, magnesium silicate, talc, and calcium hydrogen phosphate.

The glidant in the composition according to the present disclosure may be one or more compounds that can reduce the friction between particles, improve the fluidity of powder and help reduce weight differences. The glidant includes, but is not limited to, talcum powder and silicon dioxide.

The lubricant in the composition according to the present disclosure may be one or more compounds that can reduce the friction between a material and a die wall and ensure the smooth progress of pushing for tablets, filling for capsules or sub-packaging for granules. The lubricant includes, but is not limited to, magnesium stearate, stearic acid, calcium stearate, sodium stearyl fumarate, polyethylene glycol, hydrogenated vegetable oil, polyethylene glycol and sodium dodecyl sulfate.

The surfactant in the composition according to the present disclosure refers to one or more compounds that can improve the wettability and/or promote dissolution of the formulation. The surfactant includes, but is not limited to, poloxamer, sodium dodecyl sulfate, Tween and Span.

The colorant in the composition according to the present disclosure refers to one or more compounds capable of imparting a desired color to the formulation prepared from the composition. The colorant includes, but is not limited to, amaranth red, carmine red, erythrosine red, new red, tartrazine, sunset yellow, quinoline yellow, indigo, brilliant blue, beet red, shellac red, cowberry red, capsicum red, and red rice red.

The flavoring agent in the composition according to the present disclosure refers to one or more compounds used to improve or shield the bad smell and taste of drugs. The flavoring agent includes, but is not limited to, sucrose, mannitol, sorbitol, sodium citrate, aspartame, peppermint oil, essence, sodium alginate and gum arabic.

In some specific embodiments of the present disclosure, the SGLT-2 inhibitor and the angiotensin II receptor antagonist are mixed:
(1) by directly mixing; or
(2) as an internal component or an external component separately; or
(3) as an internal component simultaneously.

In some specific embodiments of the present disclosure, the angiotensin II receptor antagonist is used as an internal component, and the SGLT-2 inhibitor is used as an external component.

A method for preparing the drug is further provided according to the present disclosure, which comprises directly mixing raw and auxiliary materials.

A method for preparing the drug is further provided according to the present disclosure, which comprises the steps of mixing, granulating, sizing and blending.

A method for preparing the drug is further provided according to the present disclosure, which comprises the steps of mixing, granulating, drying, sizing and blending.

Moreover, in the method for preparing the drug according to the present disclosure, the SGLT-2 inhibitor and the angiotensin II receptor antagonist are mixed:
(1) by directly mixing; or
(2) as an internal component or an external component separately; or
(3) as an internal component simultaneously.

In some specific embodiments of the present disclosure, in the method for preparing the drug according to the present disclosure, the angiotensin II receptor antagonist is used as an internal component, and the SGLT-2 inhibitor is used as an external component.

Use of the drug or a drug prepared by the method in the manufacture of a medicament for treating essential hypertension, type 2 diabetic nephropathy associated with hypertension, diabetes, insulin resistance, hyperglycemia, hyperinsulinemia, elevated level of fatty acid or glycerol in blood, hyperlipidemia, dyslipidemia, obesity, or complications of diabetes is further provided according to the present disclosure.

The composition of an SGLT-2 inhibitor and an angiotensin II receptor antagonist at a fixed dose is provided according to the present disclosure. The composition is used for the treatment of diabetes with hypertension, while benefiting kidneys, preventing or delaying the deterioration of renal failure in patients with chronic kidney disease (CKD) and preventing cardiovascular (CV) and renal death. In addition, the compound composition at a fixed dose according to the present disclosure has good synergy and good stability, is convenient to carry or administer, significantly reduces medication cost, avoids the risk of excessive and missing administration, and improves the medication compliance of patients.

### BRIEF DESCRIPTION OF DRAWINGS

In order to more clearly illustrate the technical solutions in the examples of the present disclosure or in the prior art, the drawings required in the examples or the prior art will be briefly introduced below.

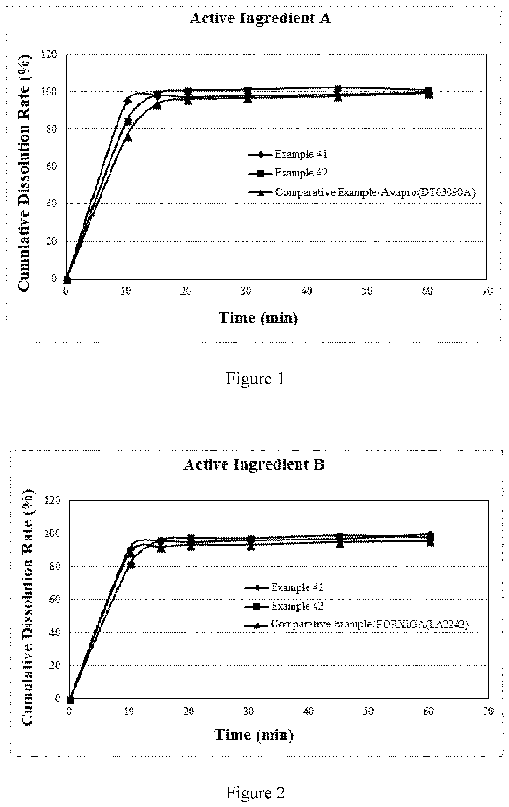

Figure 1 shows a cumulative dissolution rate (%) of active ingredient A in hydrochloric acid solution with pH1.0; and
Figure 2 shows a cumulative dissolution rate (%) of active ingredient B in hydrochloric acid solution with pH1.0.

### DETAILED DESCRIPTION

A compound composition of a sodium-glucose co-transporter 2 (SGLT-2) inhibitor and an angiotensin receptor blocker (ARB) at a fixed dose and use thereof are disclosed according to the present disclosure. Those skilled in the art can implement the present disclosure by learning from the content herein and appropriately improving the process parameters. It should be particularly noted that all similar substitutions and modifications are obvious to those skilled in the art, and are considered to be included in the present disclosure. Although the method and use of the present disclosure have been described through the preferred embodiments, it is obvious for relevant persons that the method and use described herein may be changed or appropriately modified and combined without departing from the content, spirit and scope of the present disclosure to achieve and apply the technique of the present disclosure.

The raw and auxiliary materials used in the compound composition of a sodium-glucose co-transporter 2 (SGLT-2) inhibitor and an angiotensin receptor blocker (ARB) at a fixed dose and use thereof according to the present disclosure can be purchased from the market.

Lactose (FOREMOST), mannitol (ROQUETTE), sucrose (Sichuan Boliheng), calcium carbonate (Shanghai Nuocheng), magnesium oxide (Hebei Baxter), crospovidone (BASF), cross-linked sodium carboxymethyl cellulose (Asahi Kasei), hydroxypropyl methylcellulose (Dow Chemical), microcrystalline cellulose (JRS), silicon dioxide (Huzhou Zhanwang), magnesium stearate (Huzhou Zhanwang), low-substituted hydroxypropyl cellulose (Shin-Etsu Chemical), hydroxypropyl cellulose (Nippon Soda Co., Ltd.), pregelatinized starch (Asahi Kasei), talcum powder (Guangxi Longsheng Huamei), sodium stearyl fumarate (Shanghai Chineway), gelatin hollow capsule (Suzhou Capsugel Co., Ltd.), hydroxypropyl methylcellulose hollow capsule (Suzhou Capsugel Co., Ltd.).

Hereinafter, the present disclosure will be further described in conjunction with examples.

### Examples 1-15

**Table 1 Formulation Composition in Weight Percentage (%)**

| Component | Example 1 | Example 2 | Example 3 | Example 4 | Example 5 | Example 6 |
|---|---|---|---|---|---|---|
| Active ingredient A | 50.0 | 50.0 | 50.0 | 50.0 | 50.0 | 50.0 |
| Active ingredient B | 4.1 | 4.1 | 4.1 | 4.1 | 4.1 | 4.1 |
| Lactose | 21.7 | / | / | / | / | 21.7 |
| Mannitol | / | 21.7 | / | / | / | / |
| Sucrose | / | / | 21.7 | / | / | / |
| Calcium carbonate | / | / | / | 21.7 | / | / |
| Magnesium oxide | / | / | / | / | 21.7 | / |
| Crospovidone | 7.5 | 7.5 | 7.5 | 7.5 | 7.5 | / |
| Cross-linked sodium carboxymeth yl cellulose | / | / | / | / | / | 7.5 |
| Hydroxyprop yl methylcellulo se | 2.0 | 2.0 | 2.0 | 2.0 | 2.0 | 2.0 |
| Microcrystall ine cellulose | 9.7 | 9.7 | 9.7 | 9.7 | 9.7 | 9.7 |
| Silicon dioxide | 4.0 | 4.0 | 4.0 | 4.0 | 4.0 | 4.0 |
| Magnesium stearate | 1.0 | 1.0 | 1.0 | 1.0 | 1.0 | 1.0 |
| Total | 100.0 | 100.0 | 100.0 | 100.0 | 100.0 | 100.0 |

**Table 2 Formulation Composition in Weight Percentage (%)**

| Component | Example 7 | Example 8 | Example 9 | Example 10 | Example 11 | Example 12 |
|---|---|---|---|---|---|---|
| Active ingredient A | 50.0 | 50.0 | 50.0 | 50.0 | 50.0 | 50.0 |
| Active ingredient B | 4.1 | 4.1 | 4.1 | 4.1 | 4.1 | 4.1 |
| Lactose | 21.7 | 21.7 | 21.7 | 21.7 | 21.7 | 21.7 |
| Crospovidone | / | 7.5 | 7.5 | 7.5 | 7.5 | 7.5 |
| Low-substitut ed hydroxyprop yl cellulose | 7.5 | / | / | / | / | / |
| Hydroxyprop yl methylcellulo se | 2.0 | / | 2.0 | 2.0 | 2.0 | 2.0 |
| Hydroxyprop yl cellulose | / | 2.0 | / | / | / | / |
| Microcrystall ine cellulose | 9.7 | 9.7 | / | 9.7 | 9.7 | 9.7 |
| Pregelatinize d starch | / | / | 9.7 | / | / | / |
| Silicon dioxide | 4.0 | 4.0 | 4.0 | / | 4.0 | 4.0 |
| Talcum powder | / | / | | 4.0 | 1.0 | / |
| Magnesium stearate | 1.0 | 1.0 | 1.0 | 1.0 | / | / |
| Sodium stearyl fumarate | / | / | / | / | / | 1.0 |
| Total | 100.0 | 100.0 | 100.0 | 100.0 | 100.0 | 100.0 |

### Preparation process:

The raw and auxiliary materials were weighed based on the formulation amount and mixed well. The mixed powder of each example was wrapped separately in a clean weighing paper, sealed in an aluminum foil bag, and placed at 50°C for 10 days. Samples were then taken, determined for related substances and compared with the comparative example. Since active ingredient B was relatively stable with no increase in impurities after placed under the influencing factor, only the related substances of active ingredient A are listed herein.

**Table 3 Formulation Composition in Weight Percentage (%)**

| Component | | Example 13 | Example 14 | Example 15 |
|---|---|---|---|---|
| Internal | Active ingredient A | 50 | 44.8 | 32.5 |
| | Lactose | 17 | 9.0 | 11.0 |
| | Cross-linked sodium carboxymethyl cellulose | 2 | / | / |
| | Crospovidone | / | 1.8 | 1.3 |
| | Hydroxypropyl methylcellulose | 2 | 1.8 | 1.1 |
| | Sucrose | / | 9.0 | / |
| External | Active ingredient B | 2.0 | 3.7 | 2.7 |
| | Microcrystalline cellulose | 22.0 | 23.2 | 37.1 |
| | Lactose | / | / | 10.8 |
| | Cross-linked sodium carboxymethyl cellulose | 2 | / | / |
| | Crospovidone | / | / | 2.2 |
| | Silicon dioxide | 2 | 6.0 | 0.8 |
| | Magnesium stearate | 1 | 0.9 | 0.5 |
| Total | | 100.0 | 100.0 | 100.0 |

### Preparation process:

Example 13: The internal raw and auxiliary materials were weighed based on the formulation amount of 100 granules, mixed for 15 min in a bag, then added with purified water to prepare into soft materials, granulated with a 40-mesh sieve, or dried at 50°C, and sized with a 40-mesh sieve, where the moisture content was controlled at 2% or below. The external raw and auxiliary materials were weighted at a converted amount, mixed for 10 min in a bag, and packaged into gelatin capsules.

Example 14: The internal raw and auxiliary materials were weighed based on the formulation amount of 50 tablets, mixed well, prepared into soft materials with purified water, granulated with a 80-mesh sieve, dried at 40°C, and sized with a 80-mesh sieve, where the moisture content was controlled at 2% or below. The external raw and auxiliary materials were weighted at a converted amount, passed through a 80-mesh sieve, mixed for 8 times, then added with corresponding dry particles, mixed well, and tableted.

Example 15: The internal raw and auxiliary materials were weighed based on the formulation amount of 50 granules, mixed well, prepared into soft materials with purified water, granulated with a 40-mesh sieve, dried at 50°C, and sized with a 40-mesh sieve. The external raw and auxiliary materials were weighted at a converted amount, mixed well, granulated with a 30-mesh sieve by the dry method. The internal and external raw and auxiliary materials were mixed well in proportion, and packaged into hydroxypropyl methylcellulose capsules.

**Table 4 Determination Results of Related Substances of Active Ingredient A (%)**

| Time | Example/Comparative example | RRT 0.85 | Total impurities |
|---|---|---|---|
| Day 0 | Example 1 | Not detected | 0.036 |
| | Example 2 | Not detected | 0.023 |
| | Example 3 | Not detected | 0.029 |
| | Example 4 | Not detected | 0.052 |
| | Example 5 | Not detected | 0.041 |
| | Example 6 | Not detected | 0.025 |
| | Example 7 | Not detected | 0.039 |
| | Example 8 | Not detected | 0.038 |
| | Example 9 | Not detected | 0.028 |
| | Example 10 | Not detected | 0.052 |
| | Example 11 | Not detected | 0.040 |
| | Example 12 | Not detected | 0.063 |
| | Example 13 | Not detected | 0.046 |
| | Example 14 | Not detected | 0.052 |
| | Example 15 | Not detected | 0.053 |
| | Comparative example/Avapro(DT04448)^{Note 1} | 0.034 | 0.119 |
| 50°C for 10 days | Example 1 | Not detected | 0.026 |
| | Example 2 | Not detected | 0.029 |
| | Example 3 | Not detected | 0.034 |
| | Example 4 | Not detected | 0.032 |
| | Example 5 | Not detected | 0.028 |
| | Example 6 | Not detected | 0.015 |
| | Example 7 | Not detected | 0.031 |
| | Example 8 | Not detected | 0.032 |
| | Example 9 | Not detected | 0.031 |
| | Example 10 | Not detected | 0.051 |
| | Example 11 | Not detected | 0.030 |
| | Example 12 | Not detected | 0.031 |
| | Example 13 | Not detected | 0.049 |
| | Example 14 | Not detected | 0.057 |
| | Example 15 | Not detected | 0.056 |
| | Comparative example/ Avapro(DT04448) | 0.074 | 0.165 |

| | | | |
|---|---|---|---|
| Note 1: Avapro is a commercial reference product for active ingredient A single tablet, with a batch number of DT04448. | | | |

Conclusion: The results of related substances of active ingredient A show that the comparative example at RRT 0.85 had an increase in impurities after being placed at high temperature, while Examples 1-15 had no obvious increase in impurities after being placed at high temperature, and the total impurities were also less.

### Examples 16-18

**Table 5 Formulation Composition of Unit Formulation (mg/tablet)**

| Component | | Example 16 | Example 17 | Example 18 |
|---|---|---|---|---|
| Internal | Active ingredient A | 150 | 150 | 150 |
| | Active ingredient B | 12.3 | 12.3 | 12.3 |
| | Lactose | 30.75 | 0 | 30.75 |
| | Pregelatinized starch | 37.7 | 0 | 0 |
| | Microcrystalline cellulose PH101 | / | 66.95 | 22.7 |
| | Cross-linked sodium carboxymethyl cellulose | 7.5 | / | / |
| | Crospovidone | / | 7.5 | 15 |
| | Poloxamer 188 | 9 | 9 | 9 |
| | Silicon dioxide | 6 | 6 | 6 |
| | Magnesium stearate | / | 3 | 1.5 |
| External | Microcrystalline cellulose PH 102 | 34 | 34 | 34 |
| | Cross-linked sodium carboxymethyl cellulose | 7.5 | / | / |
| | Crospovidone | / | 7.5 | 15 |
| | Silicon dioxide | 2.25 | 2.25 | 2.25 |
| | Magnesium stearate | 3 | 1.5 | 1.5 |
| Total weight | | 300 | 300 | 300 |

### Preparation process:

Active ingredient B, anhydrous lactose, cross-linked sodium carboxymethyl cellulose, poloxamer 188 and silicon dioxide were weighed based on the formulation amount of 50 tablets, passed through a 65-mesh sieve, and mixed for 6 times. Then active ingredient A and pregelatinized starch were added, passed through a 65-mesh sieve, and mixed for 8 times. Granulation was performed by the dry method. The external auxiliary materials were weighted at a converted amount, mixed well and tableted. The dissolution rate was investigated in hydrochloric acid medium with pH1.0 in 15 min. Dissolution method: hydrochloric acid solution with pH1.0, paddle method, medium volume of 900 ml, temperature of 37°C, and rotation speed of 50 rpm. Determination method: Both the reference formulations of active ingredient A and active ingredient B single tablets have a dissolution rate of above 85% in 15 min, and the dissolution requirements of the examples are consistent with those of the reference formulation, so the target value is also above 85%.

### Examples 19-20

**Table 6 Formulation Composition of Unit Formulation (mg/tablet)**

| Component | | Example 19 | Example 20 |
|---|---|---|---|
| Internal | Active ingredient A | 150 | 150 |
| | Active ingredient B | 12.3 | 12.3 |
| | Lactose | 72.7 | 72.7 |
| | Cross-linked sodium carboxymethyl cellulose | 7.5 | 7.5 |
| | Hydroxypropyl methylcellulose | 6 | 6 |
| | Silicon dioxide | 1.5 | 1.5 |
| | Magnesium stearate | 0 | 1.5 |
| External | Microcrystalline cellulose PH102 | 38 | 38 |
| | Cross-linked sodium carboxymethyl cellulose | 7.5 | 7.5 |
| | Silicon dioxide | 1.5 | 1.5 |
| | Magnesium stearate | 3 | 1.5 |
| Total weight | | 300 | 300 |

### Preparation process:

The internal raw and auxiliary materials were weighed based on the formulation amount of 50 tablets, sieved and mixed well. In Example 19, the materials were prepared into soft materials with 10% hydroxypropyl methylcellulose aqueous solution, granulated with a 65-mesh sieve, dried at 40°C, sized with a 65-mesh sieve, added with the external auxiliary materials at a converted amount, and mixed well. In Example 20, the materials were granulated by the dry method, added with the external auxiliary materials at a converted amount, and mixed well. The dissolution rate was determined in hydrochloric acid medium with pH1.0 in 15 min. Determination method: paddle method, medium volume of 1000 ml, temperature of 37°C, and rotation speed of 50 rpm. Unless otherwise specified, the dissolution performed in the following examples was determined by this method.

### Examples 21-24

**Table 7 Formulation Composition of Unit Formulation (mg/tablet)**

| Component | | Example 21 | Example 22 | Example 23 | Example 24 |
|---|---|---|---|---|---|
| Internal | Active ingredient A | 150 | 150 | 150 | 150 |
| | Lactose | 72.7 | 65.2 | 65.2 | 51 |
| | Cross-linked sodium carboxymethyl cellulose | 15 | / | / | / |
| | Crospovidone | / | 7.5 | 7.5 | 20 |
| | Hydroxypropyl methylcellulose | 2 | 9 | 7.5 | 12 |
| External | Active ingredient B | 12.3 | 12.3 | 12.3 | 12.3 |
| | Microcrystalline cellulose PH102 | 25 | / | / | / |
| | Microcrystalline cellulose PH112 | / | 32 | 15.5 | 30.7 |
| | Crospovidone | / | 15 | 15 | 6 |
| | Silicon dioxide | 20 | 6 | 24 | 15 |
| | Magnesium stearate | 3 | 3 | 3 | 3 |
| Total weight | | 300 | 300 | 300 | 300 |

### Preparation process:

The internal raw and auxiliary materials were weighed based on the formulation amount of 50 tablets and mixed well. In Example 21, the materials were added with purified water to prepare into soft materials, granulated with a 40-mesh sieve, dried at 50°C, sized with a 40-mesh sieve, added with the external auxiliary materials at a converted amount, mixed in a bag for 10 min, and tableted. The mixed materials, in Example 22, were added with hydroxypropyl methylcellulose aqueous solution to prepare into soft materials and, in Examples 23 and 24, were added with purified water to prepare into soft materials, granulated with a 80-mesh sieve, dried at 40°C, and sized with a 80-mesh sieve, where the moisture content was controlled at 2% or below. The external raw and auxiliary materials were weighted at a converted amount, passed through a 80-mesh sieve, mixed for 8 times, then added with corresponding dry particles, mixed well, and tableted. The dissolution rate was determined in hydrochloric acid medium with pH1.0 in 15 min.

**Table 8 Dissolution Rate Results of Examples 16-24**

| Dissolution Rate (%) | Active ingredient A | Active ingredient B |
|---|---|---|
| Example 16 | 94.2 | 93.5 |
| Example 17 | 93.7 | 87.0 |
| Example 18 | 93.8 | 94.7 |
| Example 19 | 95.5 | 92.8 |
| Example 20 | 95.6 | 93.0 |
| Example 21 | 99.0 | 94.6 |
| Example 22 | 89.8 | 86.2 |
| Example 23 | 97.7 | 90.0 |
| Example 24 | 91.0 | 88.8 |
| Comparative Example/Avapro(8A430) | 92.6 | / |
| Comparative Example/FORXIGA (KJ3245) | / | 97.4 |

Conclusion: The dissolution rates in 15 min of active ingredient A and active ingredient B of each example were more than 85%, consistent with the dissolution rate of the corresponding comparative example.

### Examples 25-30

**Table 9 Formulation Composition of Unit Formulation (mg/tablet)**

| Component | | Example 25 | Example 26 | Example 27 | Example 28 | Example 29 | Example 30 |
|---|---|---|---|---|---|---|---|
| Internal | Active ingredient A | 150 | 75 | 150 | 300 | 75 | 300 |
| | Lactose | 51 | 25.5 | 51 | 102 | 25.5 | 102 |
| | Crospovid one | 6 | 3 | 6 | 12 | 3 | 12 |
| | Hydroxypr opyl methylcell ulose | 6 | 3 | 6 | 12 | 3 | 12 |
| External | Active ingredient B | 12.3 | 6.15 | 6.15 | 12.3 | 12.3 | 6.15 |
| | Microcryst alline cellulose | 53.7 | 26.85 | 59.85 | 119.7 | 20.7 | 125.85 |
| | Crospovid one | 6 | 3 | 6 | 12 | 3 | 12 |
| | Silicon dioxide | 12 | 6 | 12 | 24 | 6 | 24 |
| | Magnesiu m stearate | 3 | 1.5 | 3 | 6 | 1.5 | 6 |
| Total weight | | 300 | 150 | 300 | 600 | 150 | 600 |

### Preparation process:

Active ingredient A, cross-linked povidone, lactose monohydrate and hydroxypropyl methylcellulose were weighed based on the formulation amount, mixed well, added with purified water to prepare into soft materials, granulated with a 80-mesh sieve, dried at 40°C, sized with a 80-mesh sieve, where the moisture content was controlled at 2% or below. The external raw and auxiliary materials were weighted at a converted amount, passed through a 80-mesh sieve, mixed for 8 times, then added with dry particles, mixed well, and tableted. The dissolution rate was determined in hydrochloric acid medium with pH1.0 in 15 min.

**Table 10 Dissolution Rate Results of Examples 25-30**

| Dissolution Rate (%) | Active ingredient A | Active ingredient B |
|---|---|---|
| Example 25 | 99.0 | 95.8 |
| Example 26 | 98.5 | 88.9 |
| Example 27 | 98.6 | 96.4 |
| Example 28 | 93.5 | 95.2 |
| Example 29 | 100.6 | 88.0 |
| Example 30 | 95.8 | 92.1 |
| Comparative Example/ Avapro (ET01506, Specification: 75 mg) | 90.0 | / |
| Comparative Example/ Avapro (DT04448, Specification: 150 mg) | 92.6 | / |
| Comparative Example/ Avapro(DT03090A, Specification: 300 mg) | 93.2 | / |
| Comparative Example/ FORXIGA(LB0143, Specification: 5 mg) | / | 92.1 |
| Comparative Example/ FORXIGA(KJ3245, Specification: 10 mg) | / | 91.8 |

### Examples 31-33

**Table 11 Formulation Composition of Unit Formulation (mg/tablet)**

| Component | | Example 31 | Example 32 | Example 33 |
|---|---|---|---|---|
| Internal | Active ingredient A | 300 | 300 | 300 |
| | Active ingredient B | / | / | 12.3 |
| | Lactose | 102 | 102 | 102 |
| | Cross-linked sodium carboxymethyl cellulose | / | 12 | 12 |
| | Crospovidone | 12 | / | / |
| | Hydroxypropyl methylcellulose | 10 | / | 8 |
| | Hydroxypropyl cellulose | / | 24 | / |
| External | Active ingredient B | 12.3 | 12.3 | / |
| | Microcrystalline cellulose | 31.7 | 119.7 | 329.7 |
| | Lactose | / | / | / |
| | Cross-linked sodium carboxymethyl cellulose | / | 12 | 20 |
| | Crospovidone | 15 | / | / |
| | Silicon dioxide | 12 | 12 | 8 |
| | Magnesium stearate | 5 | 6 | 8 |
| Total weight | | 500 | 600 | 800 |

### Preparation process:

Example 31: The internal raw and auxiliary materials were weighed based on the formulation amount of 100 tablets, mixed well, prepared into soft materials with purified water, granulated with a 40-mesh sieve, dried at 50°C, sized with a 40-mesh sieve, added with the external raw and auxiliary materials at a converted amount, mixed well, and tableted.

Example 32: The internal raw and auxiliary materials were weighed based on the formulation amount of 25 tablets, mixed well, prepared into soft materials with purified water, granulated with a 40-mesh sieve, dried at 60°C, sized with a 40-mesh sieve, added with the external raw and auxiliary materials at a converted amount, mixed well, and tableted.

Example 33: The internal raw and auxiliary materials were weighed based on the formulation amount of 25 tablets, mixed well, prepared into soft materials with purified water, granulated with a 40-mesh sieve, dried at 40°C, sized with a 40-mesh sieve, added with the external raw and auxiliary materials at a converted amount, mixed well, and tableted.

The dissolution rate of each example was determined in hydrochloric acid medium with pH1.0 in 15 min. The results were as follows:

**Table 12 Dissolution Rate Results of Examples 31-33**

| Dissolution Rate (%) | Active ingredient A | Active ingredient B |
|---|---|---|
| Example 31 | 97.1 | 94.8 |
| Example 32 | 89.7 | 86.5 |
| Example 33 | 86.7 | 87.9 |

### Examples 34-40

**Table 13 Formulation Composition of Unit Formulation (mg/tablet)**

| Component | | Example 34 | Example 35 | Example 36 | Example 37 |
|---|---|---|---|---|---|
| First layer | Telmisartan | 40 | / | / | / |
| | Losartan potassium | / | 50 | / | / |
| | Candesartan | / | / | 8 | / |
| | Azilsartan | / | / | / | 40 |
| | Sodium hydroxide | 3.36 | / | / | 0.69 |
| | Povidone | 12 | / | / | / |
| | Meglumine | 12 | / | / | / |
| | Sorbitol | 168.64 | / | / | / |
| | Microcrystalline cellulose | / | 52.5 | / | 18 |
| | Lactose monohydrate | / | 25.5 | 89.4 | / |
| | Pregelatinized starch | / | 20.95 | / | / |
| | Calcium carboxymethyl cellulose | / | / | 5.6 | / |
| | Hydroxypropyl methylcellulose | / | / | 4 | / |
| | Corn starch | / | / | 20 | / |
| | Polyethylene glycol | / | / | 2.6 | / |
| | Mannitol | / | / | / | 95.63 |
| | Fumaric acid | / | / | / | 2 |
| | Hydroxyproline | / | / | / | 5.4 |
| | Cross-linked sodium carboxymethyl cellulose | / | / | / | 13.8 |
| | Magnesium stearate | 4 | 1.05 | 0.4 | 1.8 |
| Second layer | Active ingredient B | 12.3 | / | 12.3 | 12.3 |
| | Canagliflozin | / | 300 | / | / |
| | Microcrystalline cellulose | 171.45 | 242.9 | 171.45 | 171.45 |
| | Anhydrous lactose | 50 | 100 | 50 | 50 |
| | Crospovidone | 10 | 30 | 10 | 10 |
| | Silicon dioxide | 3.75 | 20 | 3.75 | 3.75 |
| | Magnesium stearate | 2.5 | 5 | 2.5 | 2.5 |

**Table 14 Formulation Composition of Unit Formulation (mg/tablet)**

| Component | | Example 38 | Example 39 | Example 40 |
|---|---|---|---|---|
| First layer | Valsartan | 40 | / | / |
| | Olmesartan | / | 20 | / |
| | Active ingredient A | / | / | 300 |
| | Hydroxypropyl methylcellulose | / | / | 10 |
| | Microcrystalline cellulose | 72.5 | 128 | / |
| | Lactose monohydrate | 30 | 40 | 102 |
| | Cross-linked sodium carboxymethyl cellulose | / | 10 | 12 |
| | Cross-linked povidone | 6 | / | / |
| | Magnesium stearate | 1.5 | 2 | 5 |
| Second layer | Active ingredient B | 12.3 | 12.3 | 12.3 |
| | Microcrystalline cellulose | 171.45 | 171.45 | 171.45 |
| | Anhydrous lactose | 50 | 50 | 50 |
| | Crospovidone | 10 | 10 | 10 |
| | Silicon dioxide | 3.75 | 3.75 | 3.75 |
| | Magnesium stearate | 2.5 | 2.5 | 2.5 |

### Preparation process:

Examples 34 and 37: a) The raw and auxiliary materials for the first layer except sodium hydroxide and magnesium stearate were weighed based on the formulation amount of 100 tablets, mixed well, and prepared into soft materials with sodium hydroxide aqueous solution, granulated with a 40-mesh sieve, dried at 40°C, sized with a 40-mesh sieve, added with magnesium stearate at a converted amount, and mixed well; b) The raw and auxiliary materials for the second layer were weighed and mixed well; and c) The blended particles of the first layer and the mixed powder of the second layer were pressed into a double-layer tablet.

Examples 35 and 36: a) The raw and auxiliary materials for the first layer were weighed based on the formulation amount of 100 tablets and mixed well; b) The raw and auxiliary materials for the second layer were weighed and mixed well; and c) The mixed powder of the first layer and the mixed powder of the second layer were pressed into a double-layer tablet.

Examples 38-40: a) Part of magnesium stearate and all other raw and auxiliary materials for the first layer were weighed based on the formulation amount of 100 tablets, mixed well, granulated by the dry method, then added with the remaining magnesium stearate, and mixed well; b) Part of silicon dioxide and magnesium stearate and all other raw and auxiliary materials for the second layer were weighed, mixed well, granulated by the dry method, then added with the remaining silicon dioxide and magnesium stearate, and mixed well; and c) The blended particles of the first layer and the mixed powder of the second layer were pressed into a double-layer tablet.

The dissolution rate of some examples was determined in hydrochloric acid medium with pH1.0 in 15 min. The results were as follows:

**Table 15 Dissolution Rate Results of Examples 39 and 40**

| Dissolution Rate (%) | Olmesartan | Active ingredient A | Active ingredient B |
|---|---|---|---|
| Example 39 | 85.6 | / | 89.8 |
| Example 40 | / | 87.3 | 90.1 |

### Effect Example

**Table 16 Formulation Composition of Unit Formulation (mg/tablet)**

| Component | | Example 41 | Example 42 |
|---|---|---|---|
| Internal | Active ingredient A | 300 | 300 |
| | Active ingredient B | / | 12.3 |
| | Lactose monohydrate | 102 | 102 |
| | Cross-linked sodium carboxymethyl cellulose | 12 | / |
| | Crospovidone | / | 12 |
| | Hydroxypropyl methylcellulose | 12 | 10 |
| External | Active ingredient B | 12.3 | / |
| | Microcrystalline cellulose | 131.7 | 54.7 |
| | Cross-linked sodium carboxymethyl cellulose | 12 | / |
| | Crospovidone | / | 12 |
| | Silicon dioxide | 12 | 12 |
| | Magnesium stearate | 6 | 5 |
| Weight of tablet core/content | | 600 | 520 |

### Preparation process:

The internal raw and auxiliary materials were weighed based on the formulation amount of 100 tablets, mixed in a bag for 15 min, added with purified water to prepare into soft materials, granulated with a 40-mesh sieve, dried at 40°C (Example 42) or 50°C (Example 41), sized with a 40-mesh sieve, where the moisture content was controlled at 2% or below. The external raw and auxiliary materials were weighted at a converted amount, mixed in a bag for 15 min, and tableted. The dissolution curve of the examples was determined in hydrochloric acid medium with pH1.0.

**Table 17 Cumulative Dissolution Rate (%) of Active Ingredient A in Hydrochloric Acid Solution with pH1.0**

| Time (min) | Cumulative Dissolution Rate (%) | | |
|---|---|---|---|
| | Example 41 | Example 42 | Comparative Example /Avapro (DT03090A) |
| 10 | 95.4 | 84.7 | 76.5 |
| 15 | 98.6 | 99.4 | 93.6 |
| 20 | 97.6 | 101.2 | 96.3 |
| 30 | 98.3 | 101.6 | 97.0 |
| 45 | 99.0 | 102.7 | 97.9 |
| 60 | 100.1 | 101.4 | 99.7 |

**Table 18 Cumulative Dissolution Rate (%) of Active Ingredient B in Hydrochloric Acid Solution with pH1.0**

| Time (min) | Cumulative Dissolution Rate (%) | | |
|---|---|---|---|
| | Example 41 | Example 42 | Comparative Example/ FORXIGA (LA2242) |
| 10 | 91.5 | 81.6 | 88.9 |
| 15 | 95.5 | 96.0 | 91.8 |
| 20 | 95.0 | 97.6 | 93.1 |
| 30 | 96.0 | 97.4 | 93.2 |
| 45 | 97.1 | 99.1 | 94.8 |
| 60 | 99.7 | 97.9 | 95.4 |

**Table 19 Determination Results of Related Substances of Active Ingredient A (%)**

| Time | Example/Comparative Example | RRT 0.85 | Total impurities |
|---|---|---|---|
| Day 0 | Example 41 | Not detected | 0.042 |
| | Example 42 | Not detected | 0.041 |
| | Comparative Example/ Avapro (DT04448) | 0.034 | 0.119 |
| 50°C for 10 days | Example 41 | Not detected | 0.045 |
| | Example 42 | Not detected | 0.048 |
| | Comparative Example/ Avapro (DT04448) | 0.074 | 0.165 |

The self-made formulations of Examples 41 and 42 and reference formulation were subjected to PK research in Beagle dogs. A 3-perid 3-crossover study was conducted by using three beagle dogs (11.6-14.8 kg), in which single reference formulation A and reference formulation B were administered simultaneously. Blood samples were taken at 0.25 h, 0.5 h, 0.75 h, 1 h, 1.5 h, 2 h, 2.5 h, 3 h, 4 h, 6 h, 8 h, 12 h and 24 h after administration, and detected by LC-MS. The results were as follows:

**Table 20 Animal Experimental Results of Active Ingredient A**

| Example | Example 41 | | Example 42 | |
|---|---|---|---|---|
| Animal Number | Cₘₐₓ Ratio | AUC₀₋₂₄ Ratio | Cₘₐₓ Ratio | AUC_{0-24 Ratio} |
| | C_{max self-made}/C_{max reference} | AUC_{0-24 self-made}/AUC₀₋₂₄ reference | C_{max self-made}/C_{max reference} | AUC_{0-24 self- made}/AUC_{0-24 reference} |
| dog-#1 | 123.21 | 107.43 | 124.61 | 121.11 |
| dog-#2 | 104.66 | 84.69 | 115.37 | 123.11 |
| dog-#3 | 94.78 | 118.27 | 115.36 | 119.38 |
| (n=3) Mean | 107.55 | 103.46 | 118.45 | 121.20 |
| SD | 14.43 | 17.14 | 5.34 | 1.87 |
| CV% | 13.4 | 16.6 | 4.5 | 1.5 |

**Table 21 Animal Experimental Results of Active Ingredient B**

| Example | Example 41 | | Example 42 | |
|---|---|---|---|---|
| Animal Number | Cₘₐₓ Ratio | AUC₀₋₂₄ Ratio | Cₘₐₓ Ratio | AUC_{0-24 Ratio} |
| | C_{max self-made}/C_{max reference} | AUC_{0-24 self-made}/AUC_{0-24 reference} | C_{max self-made}/C_{max reference} | AUC_{0-24 self- made}/AUC_{0-24 reference} |
| dog-#1 | 98.58 | 101.79 | 104.46 | 113.85 |
| dog-#2 | 119.63 | 87.14 | 101.87 | 103.95 |
| dog-#3 | 110.78 | 120.59 | 97.25 | 96.99 |
| (n=3) Mean | 109.66 | 103.17 | 101.20 | 104.93 |
| SD | 10.57 | 16.77 | 3.65 | 8.47 |
| CV% | 9.6 | 16.3 | 3.6 | 8.1 |

Conclusion: It can be seen from the above results the Cₘₐₓ Ratio and AUC₀₋₂₄ Ratio of the self-made formulation and the original formulation in beagle dogs are both in the range of 80%-125%, indicating that the self-made formulation and the original formulation are bioequivalent in beagle dogs.

The composition of an SGLT-2 inhibitor and an angiotensin receptor blocker and use thereof according to the present disclosure are described in detail above. The principle and implementation of the present disclosure are illustrated by using specific embodiments herein. The above descriptions of the embodiments are only used to facilitate understanding of the method and the core idea of the present disclosure. It should be noted that, several improvements and modifications may be made by those skilled in the art to the present disclosure without departing from the principle of the present disclosure, and these improvements and modifications also fall within the protection scope of the claims of the present disclosure.

## Claims

1. A composition, comprising an SGLT-2 inhibitor and an angiotensin II receptor antagonist.

2. The composition according to claim 1, wherein the SGLT-2 inhibitor is selected from the group consisting of dapagliflozin, canagliflozin, empagliflozin, ipragliflozin, luseogliflozin, tofogliflozin, ertugliflozin, janagliflozin, bexagliflozin, sotagliflozin, henagliflozin, tianagliflozin, remogliflozin, alligliflozin, remogliflozin etabonate, (1R,2S,3S,4R,5S)-5-(4-chloro-3-(4-ethoxybenzyl)phenyl)-1-((R)-1-hydroxyethyl)-6,8-dioxabicyc lo[3.2.1]octane-2,3,4-triol, and a mixture thereof.

3. The composition according to claim 1 or 2, wherein the angiotensin II receptor antagonist is selected from the group consisting of irbesartan, candesartan, valsartan, telmisartan, losartan, iprasartan, olmesartan, azilsartan, and a mixture thereof.

4. The composition according to any one of claims 1 to 3, wherein the SGLT-2 inhibitor is selected from the group consisting of dapagliflozin, a pharmaceutically acceptable salt, ester, cocrystal, complex or solvate of dapagliflozin, and a mixture thereof; and the angiotensin II receptor antagonist is selected from the group consisting of irbesartan, a pharmaceutically acceptable salt or solvate of irbesartan, and a mixture thereof.

5. The composition according to any one of claims 1 to 4, wherein a weight ratio of the SGLT-2 inhibitor to the angiotensin II receptor antagonist is 1:960-300:1, preferably 1:240-75:1, more preferably 1:120-1:7.5, more preferably (8-300):(2-300), and most preferably 8:300, 20:12.3, 40:12.3, 50:4.1, 50:2, 44.8:3.7, 32.5:2.7, 150:12.3, 150:6.15, 75:12.3, 300:6.15 or 300:12.3.

6. Use of the composition according to any one of claims 1 to 5 in the manufacture of a medicament for treating essential hypertension, type 2 diabetic nephropathy associated with hypertension, diabetes, insulin resistance, hyperglycemia, hyperinsulinemia, elevated level of fatty acid or glycerol in blood, hyperlipidemia, dyslipidemia, obesity, or complications of diabetes.

7. A drug, comprising the composition according to any one of claims 1 to 5 and a pharmaceutically acceptable adjuvant.

8. The drug according to claim 7, wherein the adjuvant is selected from the group consisting of a filler, binder, disintegrant, antiadherent, adsorbent, glidant, lubricant, surfactant, chelating agent, colorant, taste masking agent, and a mixture thereof.

9. The drug according to claim 7 or 8, wherein the drug is in a dosage form selected from the group consisting of a tablet, capsule, granule, suspension, film, and a mixture thereof, preferably a tablet, dry suspension, capsule or granule.

10. The drug according to any one of claims 7 to 9, wherein the SGLT2 inhibitor and the angiotensin II receptor antagonist are mixed:
(1) by directly mixing; or
(2) as an internal component or an external component separately; or
(3) as an internal component simultaneously; or
(4) by separately tableting and then pressing into a double-layer tablet.

11. The drug according to claim 10, wherein the angiotensin II receptor antagonist is used as an internal component and the SGLT-2 inhibitor is used as an external component.

12. A method for preparing the drug according to any one of claims 7 to 11, comprising directly mixing raw and auxiliary materials.

13. A method for preparing the drug according to any one of claims 7 to 11, comprising steps of mixing, granulating, sizing and blending.

14. A method for preparing the drug according to any one of claims 7 to 11, comprising steps of mixing, granulating, drying, sizing and blending.

15. Use of the drug according to any one of claims 7 to 11 or a drug prepared by the method according to any one of claims 12 to 14 in the manufacture of a medicament for treating essential hypertension, type 2 diabetic nephropathy associated with hypertension, diabetes, insulin resistance, hyperglycemia, hyperinsulinemia, elevated level of fatty acid or glycerol in blood, hyperlipidemia, dyslipidemia, obesity, or complications of diabetes.
